**Europäisches Patentamt**

**European Patent Office**

**Office européen des brevets**

(11) Publication number: **0 105 106**
**B1**

(12) **EUROPEAN PATENT SPECIFICATION**

(45) Date of publication of patent specification: **17.10.90**

(21) Application number: **83107071.9**

(22) Date of filing: **19.07.83**

(51) Int. Cl.⁵: **A 61 K 31/73** // (A61K31/73, 31:635)

(54) Anti-bacterial composition.

(30) Priority: **30.08.82 JP 150398/82**

(43) Date of publication of application:
**11.04.84 Bulletin 84/15**

(45) Publication of the grant of the patent:
**17.10.90 Bulletin 90/42**

(84) Designated Contracting States:
**DE FR GB**

(56) References cited:
**EP-A-0 055 609**
**GB-A-1 252 373**

**CHEMICAL ABSTRACTS, vol. 92, no. 22, 2nd June 1980, page 302, no. 185930u, Columbus, Ohio, US; & JP - A - 79 148 090 (AJINOMOTO CO., LTD.) 19-11-1979**

The file contains technical information submitted after the application was filed and not included in this specification

(73) Proprietor: **DAITO KOEKI KABUSHIKI KAISHA 326, Yokamachi Toyama-shi Toyama (JP)**

(72) Inventor: **Takamatsu, Takeshi 118-14, Sakuragicho Chiba-shi Chiba (JP)**
Inventor: **Kitao, Tatsuo 5265, Yoshihara Nyuzenmachi Shimoniikawa-gun Toyama (JP)**
Inventor: **Koyagi, Yasuhiro 15-105, Yamamuroaraya Toyama (JP)**

(74) Representative: **Henkel, Feiler, Hänzel & Partner Möhlstrasse 37 D-8000 München 80 (DE)**

Courier Press, Leamington Spa, England.

# EP 0 105 106 B1

**Description**

The present invention relates to anti-bacterial composition.

Sulfonamides are generally called sulfa drugs, which have been widely used for medical and agricultural applications for a long time. Particularly, sulfamethoxazol is now used alone, or as a mixture with trimethoprim.

Chitosan is produced by deacetylating chitin (a polysaccharide of N-acetyl-D-glucosamine), contained in crustaceans such as crabs and shrimps, in an alkaline aqueous solution. Chitin, as well as cellulose, has been well known for many years, but not yet in any industrial application. However, chitosan which is an alkaline hydrolyzate of chitin, owing to studies made in recent years, is applied as a botanical antivirotic, or a highly polymeric flocculating agent, a sedimentation agent and a filtration facilitator utilizing the properties of chitosan as a natural type of highly polymerized cationic compound, and further, tentatively for fixing the enzyme producing bacteria.

As the results of a thorough study on the combination of active ingredients as anti-bacterial compounds the present inventors found that the combination of sulfamethoxazole with chitosan or salts thereof can display an outstanding combined effect, leading to the present invention.

Among sulfamic acids, which are generally used as anti-bacterial agents, sulfamethoxazol is particularly effective. The commercially available sulfamethoxazol (SMX) can be used as it is.

Chitosan used according to the present invention need not be limited particularly. There is preferably used chitosan which is produced such that chitin contained in crustaceans such as crabs and shrimps is deacetylated in an alkaline aqueous solution, and not restricted by the molecular weight and the degree of acetylization. The alkaline aqueous solutions used for treating chitin include aqueous solutions of sodium hydroxide and potassium hydroxide. It is preferable to treat the shells of crabs and shrimps with hydrochloric acid or formic acid to remove an ash-content such as calcium carbonate prior to alkali treatment.

The anti-bacterial compositions according to the present invention include sulfamethoxazole and chitosan or a salt thereof. The mixing ratio therebetween is desirably selected depending on the applications of the anti-bacterial compositions. In the case of an anti-bacterial medical treatment, presupposing doses for oral administration for example, the mixing ratio between sulfamethoxazole and chitosan or a salt thereof must be selected so that the combined effect can be maximal depending on the concentrations of sulfamethoxazole and chitosan or a salt thereof upon the oral administration. In the case of an anti-bacterial medical treatment presupposing spraying treatment, the mixing ratio is desirably selected depending on the objects to be sprayed. In addition, according to the present invention the properties of chitosan as a polymer are utilized, whereby sulfamethoxazole is covered with the polymer for application.

## Example 1

Five grams of commercial chitosan (manufactured by Katakura Chikkarin Kabushiki Kaisha, a light beige-colored scale-like powder, insoluble in water) was suspended in a mixture of formic acid of 150 ml with hydrochloric acid (6N) of 100 ml, and heated in a water bath heated to a temperature of 80 ~ 90°C for about two hours while being agitated, to give a solution which was filtered. Subsequently, the filtrate was cooled, and acetone was added thereto until the total volume becomes 1,000 ml. Then, the filtrate was left to stand for 24 hours, the separated-out crystals were centrifuged, washed several times using acetone, dried in a vacuum at a temperature of 50°C, filtered through 80 mesh, and a sample of chitosan was obtained. On the other hand, commercial sulfamethoxazol (SMX) was used as the sulfonamide.

Tests for anti-bacterial properties were conducted on compositions of the sample of chitosan and SMX, mixed according to the mixing ratios shown in Tables 1 to 3.

As strains to be tested, there are used *Staphylococcus aureus* 209P (a typical example of Gram positive bacteria), *Escherichia coli* NIHJ (a typical example of Gram negative bacteria) and *Pseudomonas aeruginosa* 3456.

The combined effects obtained by the present tests were determined in accordance with the Chequer board method with reference to the disclosures of Bushby et al (Bushby, S. R. M. & G. H. Hitchings: Trimethoprim. a sulfonamide potentiator. Brit. J. pharm. Chemother *33*: 72 ~ 90, 1968) and Bohni et al (Bohni. E: Vergleichende bakteriologische Untersuchungen mit der Kombination Trimethoprim/Sulfamethoxazol in vitro und in vivo. Chemotherapy. Suppl ad. *14*: 1 ~ 21, 1969), both of whom reported the combined effect between sulfamethoxazole (SMX) and trimethoprim (TMP), both of which are commercially available at present. The results of the tests are shown in Tables 1 to 3. The areas (−) outlined and the outside areas (+) outlined in the tables exhibit respectively no growth and growth of bacterias.

2

TABLE 1

Effect on *Staphylococcus aureus*

| Chitosan (µg/mℓ) \ SMX (µg/mℓ) | 50 | 25 | 12.5 | 6.25 | 3.1 | 1.6 | 0.8 | 0.4 | 0.2 | 0.1 | 0 |
|---|---|---|---|---|---|---|---|---|---|---|---|
| 5 | − | − | − | − | − | − | − | − | − | − | − |
| 2.5 | − | − | − | − | − | + | + | + | + | + | + |
| 1.2 | − | − | − | − | − | + | + | + | + | + | + |
| 0.6 | − | − | − | − | − | + | + | + | + | + | + |
| 0.3 | − | − | − | − | + | + | + | + | + | + | + |
| 0.15 | − | − | − | − | + | + | + | + | + | + | + |
| 0.08 | − | − | − | + | + | + | + | + | + | + | + |
| 0.04 | − | − | − | + | + | + | + | + | + | + | + |
| 0.02 | − | − | − | + | + | + | + | + | + | + | + |
| 0.01 | − | + | + | + | + | + | + | + | + | + | + |
| 0 | − | + | + | + | + | + | + | + | + | + | + |

EP 0 105 106 B1

TABLE 2

Effect on *Escherichia coli*

| Chitosan (µg/mℓ) \ SMX (µg/mℓ) | 100 | 50 | 25 | 12.5 | 6.25 | 3.1 | 1.6 | 0.8 | 0.4 | 0.2 | 0 |
|---|---|---|---|---|---|---|---|---|---|---|---|
| 100 | − | − | − | − | − | − | − | + | + | + | + |
| 50 | − | − | − | − | − | − | ± | + | + | + | + |
| 25 | − | − | − | − | − | + | + | + | + | + | + |
| 12.5 | − | − | − | − | − | + | + | + | + | + | + |
| 6.25 | − | − | − | − | − | + | + | + | + | + | + |
| 3.1 | − | − | − | − | ± | + | + | + | + | + | + |
| 1.6 | − | − | − | − | + | + | + | + | + | + | + |
| 0.8 | − | − | − | + | + | + | + | + | + | + | + |
| 0.4 | − | − | − | + | + | + | + | + | + | + | + |
| 0.2 | − | − | ± | + | + | + | + | + | + | + | + |
| 0 | − | − | ± | + | + | + | + | + | + | + | + |

EP 0 105 106 B1

## TABLE 3

### Effect on *Pseudomonas aeruginosa*

| SMX (µg/mℓ) / Chitosan (µg/mℓ) | 400 | 200 | 100 | 50 | 25 | 12.5 | 6.25 | 3.1 | 1.6 | 0.8 | 0 |
|---|---|---|---|---|---|---|---|---|---|---|---|
| 200 | − | − | − | − | − | − | − | ± | + | + | + |
| 100 | − | − | − | − | − | ± | ± | + | + | + | + |
| 50 | − | − | − | − | − | ± | + | + | + | + | + |
| 25 | − | − | − | − | + | + | + | + | + | + | + |
| 12.5 | − | − | − | − | + | + | + | + | + | + | + |
| 6.25 | − | − | − | − | + | + | + | + | + | + | + |
| 3.1 | ± | + | ± | + | + | + | + | + | + | + | + |
| 1.6 | ± | + | + | + | + | + | + | + | + | + | + |
| 0.8 | ± | + | + | + | + | + | + | + | + | + | + |
| 0.4 | ± | + | + | + | + | + | + | + | + | + | + |
| 0 | + | + | + | + | + | + | + | + | + | + | + |

EP 0 105 106 B1

It is recognized from Table 1 that the minimum inhibition concentration (MIC) of SMX solely used in a test tube against the strain of *Staphylococcus aureus* is 50 µg/ml, however, when chitosan is added thereto at a rate of 2.5 µg/ml, MIC of SMX becomes 3.1 µg/ml, that is, the anti-bacterial action is increased to about 16 times SMX. The mode of anti-bacterial action as described above closely resembles the mode of anti-bacterial action of the anti-bacterial composition of SMX and trimethoprim (TMP), both of which are well known. The composition ratio of the combination chitosan and SMX is most appropriate in the ranges as given in the claims.

It is recognized from Table 2 and 3 that the combined effect of chitosan with SMX against strains of *Escherichia coli* and *Pseudomona aeruginosa* is similar to that in the case of the strain of *Staphylococcus aureus*.

### Example 2

A strain of *Escherichia coli* NIHJ is floated in physiologic saline, and a mixture solution containing the strain of *Escherichia coli* NINJ and 4% mucin, being equal in absorbance to each other is produced. ICR mice (male) were fed with 20 ~ 22 g of this mixture solution containing the bacteria. As the result, all examples of the mice infected with the strain of *Escherichia coli* (intra Peritoneal administration of 5 ~ 10 times $LD_{50}$ in mice) died within twelve hours (The first contrast group). Subsequently, 80 ~ 90% of the group of mice dosed with 5 or 10 mg of SMX in two hours after the infection were still alive after 24 hours. The rate of survival of the group of mice decreased to 10 ~ 20% after 28 hours. In contrast thereto, the group of mice orally dosed with 5 mg or 10 mg of a composition of SMX + chitosan (1:2) two hours after the infection showed a high rate of survival of 80% even after 28 hours.

### Claims

1. An antibacterial composition including chitosan or a salt thereof and sulfamethoxazole, the ratio by weight of which is
a) 50:1 to 1:5000 (for gram(+)-bacteria)
b) 62.5:1 to 1:500 (for gram(−)-bacteria)
c) 32:1 to 1:64 (for gram(+) and gram(−) bacteria).
2. An antibacterial composition according to claim 1, the ratio by weight of which is
a) 1:1.24 to 1:1250 (for gram(+) bacteria)
b) 62.5:1 to 1:62.5 (for gram(−) bacteria)
c) 32:1 to 1:64 (for gram(+) and gram(−) bacteria).

### Patentansprüche

1. Antibakterielle Zusammensetzung mit Chitosan oder einem Salz desselben und Sulfamethoxazol, im Gewichtsverhältnis
a) 50:1 bis 1:5000 (für gram(+)-Bakterien)
b) 62,5:1 bis 1:500 (für gram(−)-Bakterien)
c) 32:1 bis 1:64 (für gram(+)- und gram(−)-Bakterien).
2. Antibakterielle Zusammensetzung nach Anspruch 1, dadurch gekennzeichnet, daß das Gewichtsverhältnis
a) 1:1.24 bis 1:1250 (für gram(+)-Bakterien)
b) 62,5:1 bis 1:62,5 (für gram(−)-Bakterien)
c) 32:1 bis 1:64 (für gram(+)- und gram(−)-Bakterien)
beträgt.

### Revendications

1. Composition antibactérienne contenant du chitosan ou un de ses sels et du sulfaméthoxazole, dont le rapport pondéral est de
a) 50:1 à 1:5000 (pour les bactéries Gram(+))
b) 62,5:1 à 1:500 (pour les bactéries Gram(−))
c) 32:1 à 1:64 (pour les bactéries Gram(+) et Gram(−)).
2. Composition antibactérienne selon la revendication 1, dont le rapport pondéral est
a) 1:1,24 à 1:1250 (pour les bactéries Gram(+))
b) 62,5:1 à 1:62,5 (pour les bactéries Gram(−))
c) 32:1 à 1:64 (pour les bactéries Gram(+) et Gram(−)).